# FASCICULE DE BREVET EUROPEEN

(11) **EP 2 617 394 B1**
(45) Date de publication et mention de la délivrance du brevet: **27.05.2015**
(21) Numéro de dépôt: 13151799.7
(22) Date de dépôt: 18.01.2013
(51) Int. Cl.: A61F 2/38

(54) **Implant tibial pour une prothèse de genou unicompartimentale**
Tibialimplantat für monokondyläre Kniegelenprothese
Tibial implant for a unicompartmental knee prosthesis

(30) Priorité: 19.01.2012 FR 1250537
(43) Date de publication de la demande: 24.07.2013
(73) Titulaire: Tornier, 38330 Montbonnot-Saint-Martin (FR)
(72) Inventeur: Ball, Rob, West Olive, Michigan 49460 (US); Deschamps, Gérard, 71640 Givry (FR)
(74) Mandataire: Grand, Guillaume

(56) Documents cités:
- FR-A1- 2 686 792
- FR-A1- 2 839 442
- US-A1- 2006 235 537
- US-A1- 2009 259 314

## Description

La présente invention concerne un implant tibial pour une prothèse ou une hémiprothèse de genou unicompartimentale, ainsi qu'une prothèse de genou unicompartimentale comportant un tel implant.

Une prothèse de genou unicompartimentale est destinée à remplacer le compartiment fémoro-tibial, soit interne ou médial, soit externe ou latéral, de l'articulation du genou d'un être humain. La mise en place d'une telle prothèse est indiquée lorsqu'un seul des deux compartiments présente des dommages latéralisés du fémur et/ou du tibia. En pratique, l'implantation est souvent cimentée.

L'invention s'intéresse en particulier aux prothèses de genou unicompartimentales qui comprennent un implant fémoral, interne et/ou externe, coopérant avec un implant tibial unicompartimental, interne ou externe, incluant un corps en polyéthylène. Ces prothèses présentent de nombreux avantages, le corps en polyéthylène permettant, entre autres, de disposer d'une grande surface articulaire pour l'implant fémoral en vue d'autoriser des mouvements relatifs amples et proches de la cinématique naturelle du compartiment prothésé. Ceci étant, pour certaines sollicitations de l'articulation du genou, on constate que des conflits peuvent survenir entre l'implant tibial et les tissus mous environnants, en particulier le tendon rotulien et la capsule, ce qui induit une gène, voire des douleurs pour le patient.

A titre d'exemple, US-A-2009/0259314, sur lequel est basé le préambule de la revendication 1, divulgue un implant fémoral de ce type. En comparant les diverses figures de ce document, on remarque que la surface convexe, qui entoure la surface centrale d'appui articulaire de cet implant, en la reliant au chant périphérique de ce dernier, présente un rayon de courbure qui varie suivant la périphérie de l'implant. Ceci étant, US-A-2009/0259314 est totalement silencieux quant aux valeurs du rayon de courbure de la surface convexe précitée, l'enseignement technique de ce document étant relatif à un autre aspect de l'implant. De plus, même en considérant attentivement et en comparant les coupes des figures 3 à 8 de US-A-2009/0259314, il ne ressort pas que deux portions périphériques opposées de la surface convexe précitée seraient davantage émoussées que tout le reste de cette surface convexe.

Par ailleurs, une problématique similaire à celle évoquée plus haut se retrouve pour les hémiprothèses de genou unicompartimentales, dans lesquelles un implant tibial est prévu pour s'articuler directement contre l'un des condyles osseux du fémur.

Le but de la présente invention est de proposer une prothèse ou une hémiprothèse de genou unicompartimentale améliorée, qui, sans altérer ses performances articulaires, limite les risques de conflit entre son implant tibial et les tissus mous environnants.

A cet effet, l'invention a pour objet un implant tibial pour une prothèse ou une hémiprothèse de genou unicompartimentale, tel que défini à la revendication 1.

Elle a également pour objet une prothèse de genou unicompartimentale, telle que définie à la revendication 13.

Une des idées à la base de l'invention est de concevoir la face supérieure de l'implant tibial de manière que, sans toucher à l'essentiel de cette face supérieure, qui est constituée par une grande surface d'appui articulaire soit pour l'implant fémoral dans le cas d'une prothèse de genou, soit pour le condyle non prothésé du fémur dans le cas d'une hémiprothèse tibiale de genou, cette surface d'appui articulaire est bordée par une surface convexe dont le rayon de courbure varie suivant la périphérie de cette surface convexe de sorte que, en particulier pour des raisons liées à l'anatomie du genou, les portions périphériques antérieure et postérieure de cette surface convexe sont davantage émoussées : de cette façon, les interférences entre l'implant tibial et les tissus mous environnants, notamment le tendon rotulien et la capsule du genou, sont beaucoup moins douloureuses, voire indolores, en évitant que la face supérieure du corps de l'implant tibial ne s'appuie ou ne frotte de façon anguleuse contre les tissus mous, et ce même lorsque l'implant tibial a été implanté avec un léger débord par rapport au tibia. En dehors de ces portions antérieure et postérieure, le reste de la surface convexe, reliant la surface d'appui articulaire et le chant périphérique du corps, est moins, voire quasiment pas émoussé : autrement dit, dans les portions de la surface convexe, autres que les portions antérieure et postérieure, le rayon de courbure de cette surface convexe est petit, voire quasiment nul, ce qui maximise l'étendue de la surface d'appui articulaire au niveau de ces autres portions, en particulier au niveau de la portion latérale pour un implant tibial externe et au niveau de la partie médiale pour un implant tibial interne, dans lesquelles les contraintes de conception liées au mouvement relatif admissible entre l'implant tibial et le condyle fémoral associé sont plus fortes que les contraintes liées aux risques d'interférences avec les tissus mous immédiatement adjacents. En pratique, la réalisation de la surface convexe de la face supérieure du corps de l'implant tibial selon l'invention est facile à mettre en oeuvre, typiquement par un usinage ad hoc d'un bloc de polyéthylène constituant le corps.

Des caractéristiques additionnelles avantageuses de l'implant tibial conforme à l'invention sont spécifiées aux revendications dépendantes 2 à 12.

L'invention sera mieux comprise à la lecture de la description qui va suivre, donnée uniquement à titre d'exemple et faite en se référant aux dessins sur lesquels :
- la figure 1 est une vue en perspective d'une prothèse de genou unicompartimentale conforme à l'invention ;
- la figure 2 est une vue analogue à la figure 1, montrant uniquement l'implant tibial de la prothèse ;
- la figure 3 est une vue en élévation selon la flèche III de la figure 2 ;
- la figure 4 est une coupe selon la ligne IV-IV de la figure 3 ; et
- la figure 5 est une vue en élévation selon la flèche V de la figure 3.

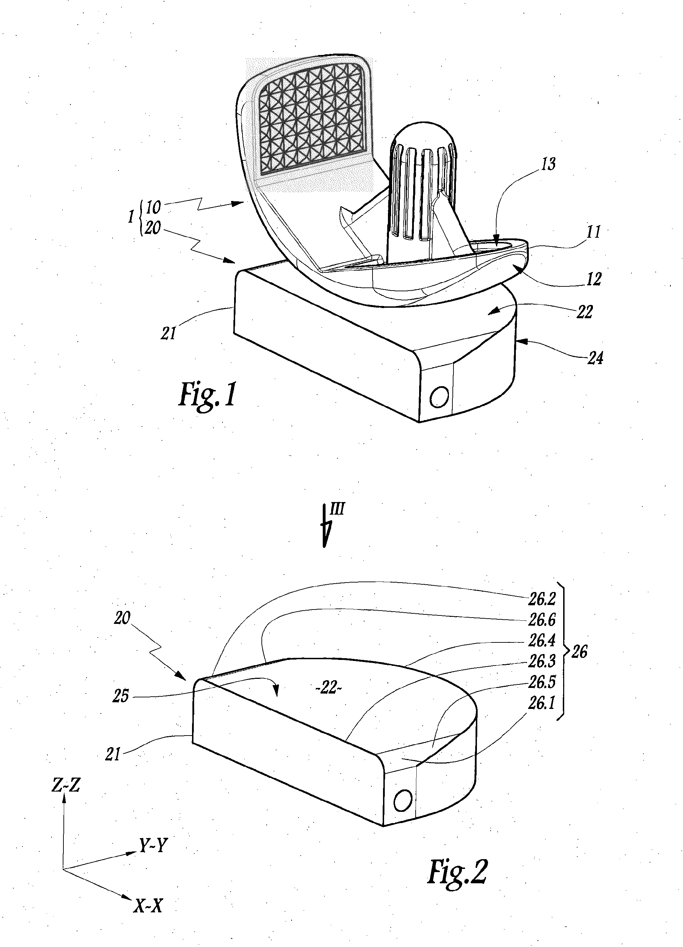

Sur la figure 1 est représentée une prothèse de genou unicompartimentale 1, comportant un implant fémoral unicompartimental 10 et un implant tibial unicompartimental 20.

L'implant fémoral 10 présente, de part et d'autre de son corps principal 11, une face convexe 12, conçue pour s'appuyer et s'articuler contre l'implant tibial 20 à des fins de restauration de l'articulation fémoro-tibiale d'un des deux compartiments du genou, et une face 13 de fixation au fémur d'un être humain, conçue pour être solidarisée en recouvrant ou en remplaçant l'un des deux condyles naturels du fémur. Le corps 11 constitue ainsi un condyle fémoral prothétique.

En pratique, diverses géométries convexes sont envisageables pour la face 12, sans que celles-ci soient limitatives de la présente invention.

La forme de réalisation de la face de fixation 13 n'est pas non plus limitative de la présente invention de sorte que cette face de fixation 13 ne sera pas décrite plus avant, étant remarqué que cette face de fixation peut aussi bien être à coupe que de resurfaçage, selon que la préparation du condyle correspondant du fémur nécessite une coupe distale ou un resurfaçage distal.

De même, le matériau constituant le corps 11 et, plus généralement, la structure, en particulier monolithique ou non, de ce corps 11 ne sont pas non plus limitatifs de la présente invention.

Avant de décrire plus avant l'implant tibial 20, on notera que, eu égard à l'agencement montré à la figure 1 entre l'implant fémoral 10 et l'implant tibial 20, notamment eu égard à l'agencement relatif d'une partie de la face de fixation 13, dédiée à la fixation sur la face distale d'un condyle du fémur, la prothèse 1 de la figure 1 est soit une prothèse pour le compartiment interne d'un genou droit, soit une prothèse pour le compartiment externe d'un genou gauche. Par commodité, la suite de la description de l'implant tibial 20 est basée sur l'hypothèse que la prothèse de genou 1 est une prothèse pour le compartiment interne d'un genou droit, ce qui implique que l'implant tibial unicompartimental 20 est un implant tibial interne, étant cependant remarqué que, comme il sera expliqué plus en détail à la fin de la présente description, cet implant tibial présente avantageusement une symétrie permettant son implantation au niveau d'autres compartiments que le compartiment interne d'un genou droit.

Comme bien visible sur les figures 2 à 5, l'implant tibial 20 comporte, voire, comme dans l'exemple considéré sur les figures, consiste en un corps massif 21. Ce corps 21 est avantageusement réalisé en polyéthylène au sens large du terme, c'est-à-dire aussi bien en polyéthylène pur, qu'en une matière plastique à base de polyéthylène, par exemple en polyéthylène haute densité (PEHD). D'autres matériaux constituant le corps 21 peuvent, en variantes, être envisagés.

Le corps 21 présente une forme volumique dont une dimension, selon un axe référencé Z-Z, est significativement plus petite que les deux autres dimensions de l'espace. Ainsi, le corps 21 délimite, d'une part, deux faces principales 22 et 23 opposées selon l'axe Z-Z, qui sont séparées l'une de l'autre par l'épaisseur, selon cet axe Z-Z, du corps 21 et, d'autre part, un chant périphérique 24, qui relie l'une à l'autre les faces 22 et 23 et dont les génératrices, dans l'exemple de réalisation considéré ici, sont rectilignes, en étant parallèles à l'axe Z-Z.

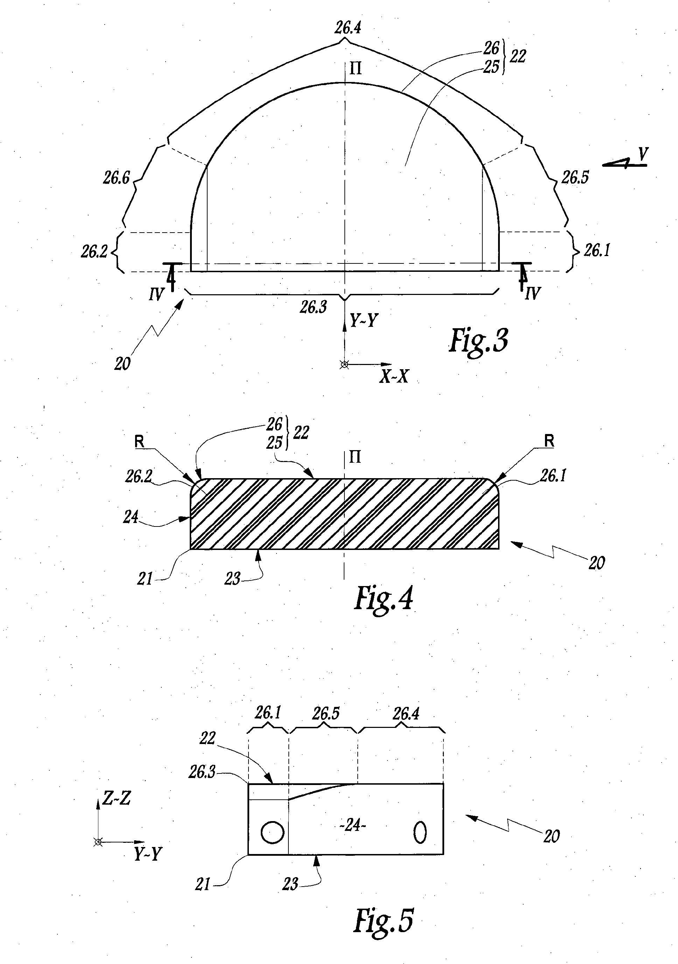

Comme bien visible sur les figures 2 et 3, le corps 21 présente, en coupe transversale à l'axe Z-Z, un contour extérieur en forme de « D ». Cette forme en « D » permet d'orienter les deux axes géométriques perpendiculaires l'un à l'autre et tous les deux orthogonaux à l'axe Z-Z : l'axe X-X est défini comme étant celui des deux axes précités qui s'étend parallèlement au côté rectiligne du profil en « D », tandis que, par voie de conséquence, l'axe Y-Y s'étend perpendiculairement au côté rectiligne précité du profil en « D », comme indiqué schématiquement sur les figures 2, 3 et 5.

La face 23 du corps 21 est, en service, destinée à être fixée à l'extrémité supérieure du tibia, en l'occurrence, pour l'exemple considéré ici, à la partie médiale de l'extrémité supérieure du tibia droit d'un patient. En pratique, la mise en oeuvre de la fixation au tibia de cette face 23, que l'on peut donc qualifier de face inférieure eu égard aux conventions anatomiques usuelles, n'est pas limitative de la présente invention : autrement dit, cette face inférieure 23 est susceptible de présenter divers aménagements ad hoc visant à assurer ou, à tout le moins, participer à la fixation du corps 21 sur le tibia. Ceci étant, selon une forme de réalisation envisageable, cette face inférieure 23 peut consister exclusivement en une surface plane dont la fixation au tibia est alors assurée par des moyens rapportés, par exemple du ciment. De même, à titre de variante non représentée, l'implant tibial selon l'invention peut, en plus du corps en polyéthylène 21, comporter une couche métallique conçue pour recouvrir la face 23 du corps 21, cette couche métallique étant adaptée, entre autres, pour intégrer des aménagement visant à renforcer, voire à assurer à eux seuls la fixation de l'implant sur le tibia : ce genre d'implant avec couche métallique inférieure est généralement qualifié de « metal back ».

La face 22 du corps 21 de l'implant tibial 20, que l'on peut donc qualifier de face supérieure par opposition à la face inférieure 23, inclut une surface plane 25 qui occupe l'essentiel de cette face supérieure 22, et ce dans la région centrale de cette dernière. En service, cette surface plane 25 forme un appui pour la face convexe 12 de l'implant fémoral 10, contre lequel cette face convexe 12 s'articule pour reproduire les comportements articulaires du compartiment correspondant du genou. Un des intérêts de prévoir plane cette surface 25 est lié au fait que les mouvements articulaires relatifs entre les implants fémoral 10 et tibial 20 ne sont pas dépendants d'un positionnement relatif strict entre les implants et sont ainsi proches de la cinématique naturelle du compartiment correspondant de l'articulation du genou. En particulier, une grande capacité de flexion de l'articulation du genou est ainsi favorisée. Ceci étant, à titre de variante non représentée, la surface 25 peut être prévue concave, notamment complémentaire d'au moins une partie de la face convexe 12 de l'implant fémoral 10.

La face supérieure 22 du corps 21 de l'implant tibial 20 inclut également une surface convexe 26, qui entoure la surface 25 et qui relie cette surface 25 au chant périphérique 24, comme bien visible sur les figures 2, 3 et 5. Cette surface convexe 26 présente un rayon de courbure qui n'est pas constant suivant la périphérie de cette surface convexe 26 : autrement dit, en coupe dans un plan à la fois parallèle à l'axe Z-Z et perpendiculaire au profil en « D » du corps 21, la surface convexe 26 présente localement une courbure qui varie lorsqu'on parcourt cette surface convexe 26 autour de la surface plane 25. Plus précisément, comme représenté sur les figures 2, 4 et 5, la surface convexe 26 inclut une portion périphérique antérieure 26.1 et une portion périphérique postérieure 26.2, qui sont opposées l'une à l'autre suivant la direction de l'axe X-X et au niveau desquelles le rayon de courbure de la surface convexe 26 présente des valeurs respectives qui sont maximales par rapport au reste de la surface convexe 26 et qui, avantageusement comme dans l'exemple de réalisation considéré ici, sont égales l'une à l'autre, en étant notées R sur la figure 4.

La surface convexe 26 inclut également une portion périphérique latérale 26.3 et une portion périphérique médiale 26.4, qui sont opposées l'une à l'autre suivant la direction de l'axe Y-Y, la portion latérale 26.3 étant située du côté rectiligne du profil en « D » du corps 21 tandis que la portion médiale 26.4 est située du côté bombé de ce profil en « D », et dont le rayon de courbure présente des valeurs respectives, avantageusement égales l'une à l'autre, qui sont minimales par rapport au reste de la surface convexe 26 et dont la valeur est significativement plus petite que la valeur R, voire est sensiblement nulle. A titre d'exemple préférentiel de dimensionnement, le rayon de courbure de la surface convexe 26 est au moins cinq fois, de préférence dix fois, plus grand dans les portions antérieure 26.1 et postérieure 26.2 que dans les portions latérale 26.3 et médiale 26.4.

En service, on comprend que la forme émoussée des portions antérieure 26.1 et postérieure 26.2 de la surface convexe 26, comparativement aux portions latérale 26.3 et médiale 26.4, limite les risques de marquage ou, plus généralement, d'interférence anguleuse avec les tissus mous environnants, situés à l'avant et à l'arrière du corps 21 de l'implant tibial 20, tels que le tendon rotulien et les segments antérieur et postérieur de la capsule du genou. Ainsi, même en cas de débord antérieur et/ou postérieur du corps 21 de l'implant tibial 20 par rapport aux faces antérieure et postérieure de l'extrémité supérieure du tibia sur laquelle l'implant 20 est fixé, la conformation moins agressive de la courbure des portions antérieure 26.1 et postérieure 26.2 de la surface convexe 26 limite, voire supprime les sensations douloureuses liées au conflit entre l'implant tibial 20 et les tissus mous précités, cette conformation s'apparentant d'ailleurs au profil anatomique des côtés antérieur et postérieur de l'extrémité supérieure du tibia.

Avantageusement, dans ses portions antérieure 26.1 et postérieure 26.2, la surface convexe 26 se raccorde tangentiellement à la surface plane 25 : cette disposition renforce le profil anatomique émoussé de la face supérieure 22 dans ses portions extrêmes antérieure et postérieure, tout en facilitant l'obtention de cette face supérieure 22, réalisée par exemple par usinage d'un bloc parallélépipédique massif de polyéthylène.

Dans le même temps, dans la portion médiale 26.4 de la surface convexe 26, au niveau de laquelle sont également adjacents des tissus mous environnants, l'étendue de la surface plane 25 est maximisée dans le sens où, dans la portion médiale de la face 22, la surface plane 25 occupe toute l'étendue disponible jusqu'au chant périphérique 24, à la dimension marginale près de la portion médiale 26.4 de la surface convexe 26.

Avantageusement, notamment pour des raisons liées à l'obtention de la face supérieure 22 du corps 21 et/ou au renforcement de la conformation anatomique de la surface convexe 26 dans ses régions périphériques immédiatement adjacentes aux portions antérieure 26.1 et postérieure 26.2, le rayon de courbure de la surface convexe 26 diminue progressivement lorsqu'on parcourt cette surface convexe 26 soit depuis la portion antérieure 26.1, soit depuis la portion postérieure 26.2, en direction de la portion médiale 26.4. Autrement dit, comme bien visible sur les figures 2 et 5, la surface convexe 26 inclut, entre ses portions antérieure 26.1 et médiale 26.4, une première portion intermédiaire 26.5, ainsi qu'une seconde portion intermédiaire entre ses portions postérieure 26.2 et médiale 26.4 : ces portions intermédiaires 26.5 et 26.6 présentent un rayon de courbure qui varie continument lorsqu'on parcourt ces portions intermédiaires suivant la périphérie de la surface convexe 26, en passant d'une valeur égale à la valeur précitée R à l'extrémité périphérique des portions intermédiaires, tournée vers les portions antérieure 26.1 et postérieure 26.2, à une valeur minimale, voire sensiblement nulle, correspondant à la valeur du rayon de courbure dans la portion médiale 26.4, à l'extrémité périphérique des portions intermédiaires, tournée vers cette portion médiale.

A titre de disposition optionnelle particulièrement avantageuse, les portions antérieure 26.1 et postérieure 26.2 de la surface convexe 26 sont symétriques l'une de l'autre par rapport à un plan π parallèle aux axes Z-Z et Y-Y, formant un plan médian vertical pour le corps 21. Cette symétrie facilite, entre autres, l'obtention de ces portions antérieure 26.1 et postérieure 26.2 de la surface convexe 26. En prévoyant avantageusement que le plan précité π constitue un plan de symétrie pour toute la face supérieure 22 du corps 21, voire pour tout ce corps 21, l'obtention de cette face 22, voire de tout le corps 21 s'en trouve encore davantage facilitée et, surtout, cela rend l'implant tibial 20 à même d'être implanté indifféremment au niveau du compartiment interne et au niveau du compartiment externe d'un genou, que ce dernier soit un genou droit ou un genou gauche. Bien entendu, selon la configuration d'implantation choisie, la portion 26.3 de la surface convexe 26, qui s'étend le long du côté rectiligne du profil en « D » du corps 21, se retrouve être soit une portion latérale, comme dans la description ci-dessus, soit une portion médiale, tandis qu'il en est inversement pour la portion opposée 26.4.

Par ailleurs, divers aménagements et variantes à l'implant tibial 20 et à la prothèse de genou unicompartimentale 1 sont envisageables. A titre d'exemple, plutôt que d'être intégré à une prothèse de genou totale telle que la prothèse 1, l'implant tibial 20 peut appartenir à, voire constituer une hémiprothèse de genou : dans ce cas, sa surface 25 forme un appui articulaire direct pour un des condyles naturels du fémur du patient, autrement dit pour un condyle fémoral non prothésé.

## Revendications

1. Implant tibial (20) pour une prothèse ou une hémiprothèse de genou unicompartimentale,
comportant un corps (21) qui présente :
- une face inférieure (23) qui est destinée à être fixée, directement ou indirectement, à l'une des parties médiale et latérale de l'extrémité supérieure du tibia,
- une face supérieure (22), qui est destinée à s'articuler contre un condyle, prothétique ou non prothésé, de l'extrémité inférieure du fémur et qui inclut une surface (25) d'appui articulaire pour ce condyle, et
- un chant périphérique (24) qui relie l'une à l'autre les faces inférieure (23) et supérieure (22),
la face supérieure (22) incluant en outre une surface convexe (26) qui entoure la surface d'appui articulaire (25) et la relie au chant périphérique (24),
**caractérisé en ce que** le rayon de courbure de la surface convexe (26) est plus grand dans des portions périphériques opposées, respectivement antérieure (26.1) et postérieure (26.2), que dans le reste de cette surface convexe.

2. Implant tibial selon la revendication 1, **caractérisé en ce que** le corps (21) est réalisé en polyéthylène.

3. Implant tibial selon la revendication 2, **caractérisé en ce que** le rayon de courbure de la surface convexe (26) est au moins cinq fois plus grand dans les portions antérieure (26.1) et postérieure (26.2) que dans le reste de la surface convexe.

4. Implant tibial selon la revendication 3, **caractérisé en ce que** le rayon de courbure de la surface convexe (26) est au moins dix fois plus grand dans les portions antérieure (26.1) et postérieure (26.2) que dans le reste de la surface convexe.

5. Implant tibial selon l'une quelconque des revendications précédentes, **caractérisé en ce que** le rayon de courbure de la surface convexe (26) est minimal, voire sensiblement nul, dans l'une des deux portions périphériques, respectivement latérale (26.3) et médiale (26.4), de cette surface convexe, qui sont opposées l'une à l'autre suivant une direction sensiblement perpendiculaire à la direction selon laquelle les portions antérieure (26.1) et postérieure (26.2) sont opposées.

6. Implant tibial selon la revendication 5, **caractérisé en ce que** le rayon de courbure de la surface convexe (26) est minimal, voire sensiblement nul, dans les deux
portions périphériques, respectivement latérale (26.3) et médiale (26.4), de cette surface convexe, qui sont opposées l'une à l'autre suivant une direction sensiblement perpendiculaire à la direction selon laquelle les portions antérieure (26.1) et postérieure (26.2) sont opposées.

7. Implant tibial selon l'une quelconque des revendications précédentes, **caractérisé en ce que**, dans ses portions antérieure (26.1) et postérieure (26.2), la surface convexe (26) se raccorde tangentiellement à la surface d'appui articulaire (25).

8. Implant tibial selon l'une quelconque des revendications précédentes, **caractérisé en ce que** les portions antérieure (26.1) et postérieure (26.2) de la surface convexe (26) sont symétriques l'une de l'autre par rapport à un plan médian (π) du corps (21), transversal aux surfaces inférieure (23) et supérieure (22).

9. Implant tibial selon la revendication 8, **caractérisé en ce que** le plan médian (π) constitue un plan de symétrie pour toute la face supérieure (22).

10. Implant tibial selon la revendication 8, **caractérisé en ce que** le plan médian
(π) constitue un plan de symétrie pour tout le corps (21)

11. Implant tibial selon l'une quelconque des revendications précédentes, **caractérisé en ce que** la surface d'appui articulaire (25) est sensiblement plane.

12. Implant tibial selon l'une quelconque des revendications précédentes, **caractérisé en ce que** l'implant tibial comporte en outre une couche métallique recouvrant la face inférieure (22) du corps (21).

13. Prothèse de genou unicompartimentale (1), comportant :
- un implant tibial unicompartimental (20) conforme à l'une quelconque des revendications précédentes, et
- un implant fémoral (10), notamment unicompartimental, qui est destiné à être fixé à l'extrémité inférieure du fémur et qui est adapté pour s'appuyer et s'articuler contre la surface d'appui articulaire (25) de la face supérieure (22) du corps (21) de l'implant tibial (20).

## Patentansprüche

1. Schienbeinimplantat (20) für eine Ein-Raum-Knieprothese oder -Halbprothese,
aufweisend einen Körper (21), der aufweist:
- eine untere Seite (23), die dazu bestimmt ist, direkt oder indirekt an einem von dem medialen oder dem lateralen Teil des oberen Schienbeinendes fixiert zu sein,
- eine obere Seite (22), die dazu bestimmt ist, sich gelenkig gegen einen Prothese- oder Nichtprothese-Gelenkkopf des unteren Oberschenkelknochenendes zu stützen und die eine Gelenkabstützfläche (25) für diesen Gelenkkopf aufweist, und
- ein Umfangsstirnseite (24), die die untere Seite (23) und die obere Seite (22) miteinander verbindet,
wobei die obere Seite (22) ferner eine konvexe Fläche (26) aufweist, die die Gelenkabstützfläche (25) umgibt und diese mit der Umfangsstirnseite (24) verbindet,
**dadurch gekennzeichnet, dass** der Krümmungsradius der konvexen Fläche (26) in gegenüberliegenden Anterior(26.1)- bzw. Posterior(26.2)-Abschnitten größer ist als im Rest dieser konvexen Fläche.

2. Schienbeinimplantat gemäß Anspruch 1, **dadurch gekennzeichnet, dass** der Körper (21) aus Polyethylene ist.

3. Schienbeinimplantat gemäß Anspruch 2, **dadurch gekennzeichnet, dass** der Krümmungsradius der konvexen Fläche (26) in dem Anterior(26.1)- und dem Posterior(26.2)-Abschnitt wenigstens fünf Mal größer ist als im Rest der konvexen Fläche.

4. Schienbeinimplantat gemäß Anspruch 3, **dadurch gekennzeichnet, dass** der Krümmungsradius der konvexen Fläche (26) in dem Anterior(26.1)- und dem Posterior(26.2)-Abschnitt wenigstens zehn Mal größer ist als im Rest der konvexen Fläche.

5. Schienbeinimplantat gemäß irgendeinem der vorhergehenden Ansprüche, **dadurch gekennzeichnet, dass** der Krümmungsradius der konvexen Fläche (26) in einem von den beiden Lateral (26.3) - bzw. Medial(26.4)-Umfangsabschnitten dieser konvexen Fläche, die sich einander in einer Richtung im Wesentlichen senkrecht zur der Richtung gegenüberliegen, in welcher sich der Anterior(26.1)- und der Posterior(26.2)-Abschnitt gegenüberliegen, minimal, wenn nicht sogar im Wesentlichen null, ist.

6. Schienbeinimplantat gemäß Anspruch 5, **dadurch gekennzeichnet, dass** der Krümmungsradius der konvexen Fläche (26) in den beiden Lateral(26.3)- bzw. Medial (26.4) - Abschnitten dieser konvexen Fläche, die sich einander in einer Richtung im Wesentlichen senkrecht zur Richtung gegenüberliegen, in welcher sich der Anterior(26.1)- und der Posterior(26.2)-Abschnitt gegenüberliegen, minimal, wenn nicht sogar im Wesentlichen null, ist.

7. Schienbeinimplantat gemäß irgendeinem der vorhergehenden Ansprüche, **dadurch gekennzeichnet, dass** sich in dem Anterior(26.1)- und dem Posterior(26.2)-Abschnitt die konvexe Fläche (26) tangential an die Gelenkabstützfläche (25) anschließt.

8. Schienbeinimplantat gemäß irgendeinem der vorhergehenden Ansprüche, **dadurch gekennzeichnet, dass** der Anterior(26.1)-und der Posterior(26.2)-Abschnitt der konvexen Fläche (26) zueinander symmetrisch sind bezüglich einer Medianebene (n) des Körpers (21) quer zu der unteren (23) und der oberen Fläche (22).

9. Schienbeinimplantat gemäß Anspruch 8, **dadurch gekennzeichnet, dass** die Medianebene (n) eine Symmetrieebene für die gesamte obere Fläche (22) bildet.

10. Schienbeinimplantat gemäß Anspruch 8, **dadurch gekennzeichnet, dass** die Medianebene (n) eine Symmetrieebene für den gesamten Körper (21) bildet.

11. Schienbeinimplantat gemäß irgendeinem der vorhergehenden Ansprüche, **dadurch gekennzeichnet, dass** die Gelenkabstützfläche (25) im Wesentlichen eben ist.

12. Schienbeinimplantat gemäß irgendeinem der vorhergehenden Ansprüche, **dadurch gekennzeichnet, dass** das Schienbeinplantat ferner aufweist eine Metallschicht, die die untere Fläche (22) des Körpers (21) bedeckt.

13. Ein-Raum-Knieprothese (1) mit:
- einem Ein-Raum-Schienbeinimplantat (20) gemäß irgendeinem der vorhergehenden Ansprüche, und
- einem Oberschenkelknochenimplantat (10), insbesondere einem Ein-Raum-Oberschenkelknochenimplantat, das dazu bestimmt ist, an einem unteren Ende des Oberschenkelknochens fixiert zu sein und das angepasst ist, um sich gegen die Gelenkabstützfläche (25) der oberen Fläche (22) des Körpers (21) des Schienbeinimplantats (20) abzustützen und gelenkzubewegen.

## Claims

1. Tibial implant (20) for a unicompartmental knee prosthesis or hemiprosthesis, comprising a body (21) having:
- a lower face (23) intended to be fixed, directly or indirectly, to one of the medial and lateral parts of the upper end of the tibia,
- an upper face (22) intended to articulate against a prosthetic or non-prosthetic condyle of the lower end of the femur and including an articular bearing surface (25) for the condyle, and
- a peripheral edge (24) connecting the lower (23) and upper (22) faces to one another,
the upper face (22) also including a convex surface (26) surrounding the articular bearing surface (25) and connecting it to the peripheral edge (24),
**characterised in that** the radius of curvature of the convex surface (26) is greater in the opposite, respectively anterior (26.1) and posterior (26.2), peripheral portions than in the rest of the convex surface.

2. Tibial implant according to claim 1, **characterised in that** the body (21) is made from polyethylene.

3. Tibial implant according to claim 2, **characterised in that** the radius of curvature of the convex surface (26) is at least five times greater in the anterior (26.1) and posterior (26.2) portions than in the rest of the convex surface.

4. Tibial implant according to claim 3, **characterised in that** the radius of curvature of the convex surface (26) is at least ten times greater in the anterior (26.1) and posterior (26.2) portions than in the rest of the convex surface.

5. Tibial implant according to any one of the preceding claims, **characterised in that** the radius of curvature of the convex surface (26) is minimal, or approximately zero, in one of the two peripheral portions, respectively lateral (26.3) and medial (26.4), of the convex surface, which are opposite one another in a direction approximately perpendicular to the direction in which the anterior (26.1) and posterior (26.2) portions are opposite.

6. Tibial implant according to claim 5, **characterised in that** the radius of curvature of the convex surface (26) is minimal, or approximately zero, in the two peripheral portions, respectively lateral (26.3) and medial (26.4), of the convex surface, which are opposite one another in a direction approximately perpendicular to the direction in which the anterior (26.1) and posterior (26.2) portions are opposite.

7. Tibial implant according to any one of the preceding claims, **characterised in that**, in its anterior (26.1) and posterior (26.2) portions, the convex surface (26) is connected tangentially to the articular bearing surface (25).

8. Tibial implant according to any one of the preceding claims, **characterised in that** the
anterior (26.1) and posterior (26.2) portions of the convex surface (26) are symmetrical with one another with respect to a median plane (n) of the body (21), transverse to the lower (23) and upper (22) surfaces.

9. Tibial implant according to claim 8, **characterised in that** the median plane (n) constitutes a plane of symmetry for the entire upper face (22).

10. Tibial implant according to claim 8, **characterised in that** the median plane (π) constitutes a plane of symmetry for the entire body (21).

11. Tibial implant according to any one of the preceding claims, **characterised in that** the articular bearing surface (25) is approximately flat.

12. Tibial implant according to any one of the preceding claims, **characterised in that** the tibial implant also comprises a metal layer covering the lower face (22) of the body (21).

13. Unicompartmental knee prosthesis (1), comprising:
- a unicompartmental tibial implant (20) according to any one of the preceding claims, and
- a femoral implant (10), in particular unicompartmental, intended to be fixed to the lower end of the femur and capable of resting and articulating against the articular bearing surface (25) of the upper face (22) of the body (21) of the tibial implant (20).
